# EUROPEAN PATENT APPLICATION

(11) **EP 3 831 277 A1**
(43) Date of publication of application: **09.06.2021**
(21) Application number: 19213217.3
(22) Date of filing: 03.12.2019
(51) Int. Cl.: A61B 5/00, A61B 5/06, A61B 5/042

(54) **REFERENCE LOCATION VISUALIZATION FOR ELECTROYPHYSIOLOGICAL MAPPING, AND ASSOCIATED DEVICES, SYSTEMS, AND METHODS**

(71) Applicant: Koninklijke Philips N.V., 5656 AG Eindhoven (NL)
(72) Inventor: IBRAGIMOV, Zalman, 5656 AE Eindhoven (NL); KARASENTI, David, 5656 AE Eindhoven (NL); SCHWARTZ, Yitzhack, 5656 AE Eindhoven (NL)
(74) Representative: Philips Intellectual Property & Standards

(57) **Abstract**

Devices, systems, and methods for visualizing a reference location of an electrophysiology device in an anatomical image are provided. According to one embodiment, an electrophysiological mapping and guidance system includes a processor circuit in communication with a catheter carrying a plurality of electrodes. The processor circuit controls the plurality of electrodes to obtain electrical measurements (e.g., voltage measurements) of an electrical field induced within an anatomical cavity. The processor circuit computes a reference location of the plurality of electrodes based on distortions in the electromagnetic field detected at a first time, computes a current location of the plurality of electrodes based on distortions in the electromagnetic field detected at a later second time, and outputs a signal to cause simultaneous display of a first visualization of the reference location and a second visualization of the current location.

## Description

### TECHNICAL FIELD

The present disclosure relates generally to electrophysiological imaging for guiding treatment procedures, and, in particular, to electrophysiological systems and methods for imaging volumes of the body and/or guiding therapy within the volumes. For example, a mapping and guidance system can include a processor circuit configured to control an electrophysiology catheter to guide a therapeutic device to a treatment site.

### BACKGROUND

Recently, there has been an increased focus on minimally invasive procedures to treat various neurological and physiological disorders, such as atrial fibrillation (AF) and hypertension. For example, AF is an abnormal heart rhythm characterized by rapid and irregular beating of the atria, and may be associated with heart palpitations, fainting, lightheadedness, shortness of breath, or chest pain. The disease is associated with an increased risk of heart failure, dementia, and stroke. AF may be caused by electrical pulses generated by secondary pacers at the ostium of the pulmonary veins. On the other hand, hypertension can be caused by renal sympathetic nerve activity (RSNA) in which feedback from overactive renal sympathetic nerves causes increased blood pressure and can contribute to the deterioration of renal function.

One way of treating such disorders includes ablating nerves and/or tissue to reduce the effect of the undesired neurological activity. Ablation can be performed in various ways, including radiofrequency (RF) ablation, ultrasonic ablation, electroporation ablation and cryoablation.

In order to guide a therapeutic device or therapeutic tool (e.g., ablation catheter, a diagnostic catheter or any endovascular tool) to the treatment site, the procedure is typically performed under external imaging, such as angiography or fluoroscopy. In conventional methods, angiographic and/or fluoroscopic procedures include injecting a contrast agent or dye into the vasculature to generate a roadmap image of the vasculature, and co-registering fluoroscopic images with the roadmap once the contrast agent has dissipated. The angiographic/fluoroscopic registration is used to show the location of the therapeutic tool, at least part of which is visible in the fluoroscopic image stream.

However, angiography/fluoroscopy image guiding techniques suffer from a number of drawbacks. For example, some patients have negative reactions to contrast agent used to generate the roadmap of the vasculature. Further, it may beneficial to limit X-ray radiation exposure to the personnel and the patients.

Another image guidance approach includes generating a map or image of an anatomy (e.g., atria of the heart, blood vessels, etc.) using electrodes to detect currents and/or voltages within the anatomy, for instance by means of an endovascular tool, such as an electrophysiology (EP) catheter.. An anatomical image is generated based on electrograms provided by the electrodes. Based on knowledge of endovascular tool and/or therapeutic tool mechanics, the physician is able to guide the endovascular tool to a location such that the therapeutic tool (e.g., ablation balloon) can be deployed at the treatment site. However, during the procedure, for number of reasons, the endovascular tool, such as the EP catheter, can move from the desired location, thereby obliging the physician to reposition the endovascular tool, which can be time consuming and may considerably delay the procedure and may generate safety issue for the patient in the event the shift/move of the endovascular device changes some parameters derived from the detected currents and/or voltages within the anatomy which are not taken into account by the physician.

### SUMMARY

Aspects of the present disclosure provide devices, systems, and methods for visualizing a reference location of an electrophysiology device in an anatomical image. For example, according to one embodiment, an electrophysiological mapping and guidance system includes a processor circuit in communication with an endovascular device, such as a catheter, carrying a plurality of electrodes. The processor circuit controls the plurality of electrodes to obtain electrical measurements (e.g., voltage measurements) of an electrical field induced within an anatomical cavity. The electrical field may be induced by the electrodes of the catheter themselves, and/or by external body patch electrodes placed on the patient's skin, wherein the emitted electrical filed will be distorted by features of the anatomy. The processor circuit tracks the location of the electrodes based on distortions of the electrical field detected by the catheter electrodes. The physician can then visualize the current location of, for instance, the endovascular device (e.g. by assessment of the location of the electrodes comprised therewith) or the electrodes by watching a display that includes a computer-generated image of the anatomical cavity and a marker representative of the real-time location of the endovascular device or the electrodes. Once the physician has determined that the electrodes are at a location of interest, the physician initiates a user input via a user input device. In response to the user input, the processor circuit designates the location of the electrodes at the time the trigger signal is received as a reference location. The processor circuit can then simultaneously output a first visualization of the current location of the electrodes and a second visualization of the designated reference location of the electrodes. In this way, if the catheter moves during the procedure, the physician can guide or have guided the catheter back to the reference location based on the first and second visualizations.

According to an exemplary embodiment, an apparatus for guiding an instrument (such as a tool, a device, a catheter or any other implement suitable to be (partially) introduced and subsequently guided into a body of a subject) within an anatomical cavity includes: a processor circuit configured for communication with a plurality of electrodes disposed on the instrument. The processor circuit is configured to: receive electrical signals from the plurality of electrodes representative of an electromagnetic field within the anatomical cavity; compute a reference location of the plurality of electrodes based on distortions in the electromagnetic field detected at a first time; compute a current location of the plurality of electrodes based on distortions in the electromagnetic field detected at a second time, wherein the second time is subsequent to the first time; and output a signal configured to cause simultaneous display of a first visualization of the reference location and a second visualization of the current location such that the current location is displayed relative to the reference location.

In some embodiments, a visual characteristic of the first visualization is different from the visual characteristic of the second visualization. In some embodiments, the visual characteristic comprises at least one of: a color, a transparency, an electrode indicium, or a pattern. In some embodiments, the second visualization, and not the first visualization, includes electrode indicia representative of relative positions of the plurality of electrodes. In some embodiments, the electrode indicia include numerical representations associated with each electrode of the plurality of electrodes.

In one aspect, the processor circuit is configured to: receive a user input indicating an instruction to compute the reference location; and compute the reference location in response to receiving the user input. In some embodiments, the processor circuit is configured to save parameters representative of the reference location to a memory in response to receiving the user input. In some embodiments, the processor circuit is further configured to generate, based on the electrical signals from the plurality of electrodes, an anatomical image of the anatomical cavity. In some embodiments, the processor circuit is configured to output the signal such that the first visualization is stationary within the anatomical image. In some embodiments, the processor circuit is further configured to: calculate a distance between the reference location and the current location of the plurality of electrodes; and output the signal to cause simultaneous display of the first visualization, the second visualization, and a third visualization of the calculated distance.

In some embodiments, the processor circuit is configured to: repeatedly compute the current location of the plurality of electrodes; and repeatedly update the second visualization to display the current location of the plurality of electrodes. In some embodiments, the processor circuit is configured to output the signal to a display in communication with the processor circuit.

According to another embodiment of the present disclosure, a system for guiding an instrument (such as a tool, a device, a catheter or any other implement suitable to be (partially) introduced and subsequently guided into a body of a subject) within an anatomical cavity, an apparatus according to any of the embodiment described herein; and the instrument. In some aspects, the instrument comprises an electrophysiology (EP) catheter comprising an elongate tip member. In another aspect, the plurality of electrodes is positioned on the elongate tip member. In other aspects, the first visualization is representative of the elongate tip member of the EP catheter at the first time. In still other aspects, the second visualization is representative of the elongate tip member of the EP catheter at the later second time.

According to another embodiment of the present disclosure, a method for guiding an instrument within an anatomical cavity includes: receiving electrical signals from a plurality of electrodes disposed on the instrument, wherein the electrical signals are representative of an electromagnetic field within the anatomical cavity; computing a reference location of the plurality of electrodes based on distortions in the electromagnetic field detected at a first time; computing a current location of the plurality of electrodes based on distortions in the electromagnetic field detected at a second time, wherein the second time is subsequent to the first time; outputting a signal configured to cause simultaneous display of a first visualization of the reference location and a second visualization of the current location such that the current location is displayed relative to the reference location.

In some embodiments, a visual characteristic of the first visualization is different from the visual characteristic of the second visualization. In some embodiments, the visual characteristic comprises at least one of: a color, a transparency, an electrode indicium, or a pattern. In some embodiments, the second visualization, and not the first visualization, includes electrode indicia representative of relative positions of the plurality of electrodes. In some embodiments, the electrode indicia include numerical representations associated with each electrode of the plurality of electrodes.

In some embodiments, the method further includes: receiving a user input indicating an instruction to compute the reference location; and computing the reference location in response to receiving the user input.

In some embodiments, the method further includes calculating a distance between the reference location and the current location of the plurality of electrodes.

In some embodiments, outputting the signal comprises outputting the signal to cause simultaneous display of the first visualization, the second visualization, and a third visualization of the calculated distance.

According to another embodiment of the present disclosure, a computer program product includes: a non-transitory computer-readable medium having program code recorded thereon, the program code including: code for receiving electrical signals from a plurality of electrodes disposed on an instrument (such as a tool, a device, a catheter or any other implement suitable to be (partially) introduced and subsequently guided into a body of a subject), wherein the electrical signals are representative of an electromagnetic field within an anatomical cavity; code for computing a reference location of the plurality of electrodes based on distortions in the electromagnetic field detected at a first time; code for computing a current location of the plurality of electrodes based on distortions in the electromagnetic field detected at a second time wherein the second time is subsequent to the first time; and code for outputting a signal configured to cause simultaneous display of a first visualization of the reference location and a second visualization of the current location such that the current location is displayed relative to the reference location.

Additional aspects, features, and advantages of the present disclosure will become apparent from the following detailed description. It will be appreciated by those skilled in the art that two or more of the above-mentioned options, implementations, and/or aspects of the invention may be combined in any way deemed useful.

### BRIEF DESCRIPTION OF THE DRAWINGS

Illustrative embodiments of the present disclosure will be described with reference to the accompanying drawings, of which:
Fig. 1 is a graphical depiction of a cryoballoon and EP catheter positioned at the PV ostium, according to aspects of the present disclosure.
Fig. 2 is a diagrammatic view of an EP-guided cryoablation system, according to aspects of the present disclosure.
Fig. 3 is a diagrammatic view of a processor circuit, according to aspects of the present disclosure.
Fig. 4 is a flow diagram of a method for visualizing a reference location in an electrophysiological mapping and/or therapy procedure, according to aspects of the present disclosure.
Fig. 5 is a graphical view of electrical signals obtained by electrodes of an EP catheter, according to aspects of the present disclosure.
Fig. 6 is a topographical view of a non-homogenous electromagnetic field within an anatomical cavity, according to aspects of the present disclosure.
Fig. 7 is a screen display of an electrophysiological mapping system, according to aspects of the present disclosure.
Fig. 8 is a screen display of an electrophysiological mapping system including a reference location visualization of an EP catheter, according to aspects of the present disclosure.
Fig. 9 is a screen display of an electro-anatomical mapping system including a reference location visualization of an EP catheter, according to aspects of the present disclosure.
Fig. 10 is a flow diagram of a method for visualizing a reference location in an electrophysiological mapping and/or therapy procedure, according to aspects of the present disclosure.
Fig. 11 is a cross-sectional view of a therapeutic balloon positioned at a stenosed segment within a vessel, according to aspects of the present disclosure.

### DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. For example, although the following disclosure may refer to embodiments that include balloon therapy procedures, cryoablation, cyroballoons, cryocatheters, balloon angioplasty, RF balloon ablation, or RF balloons, it will be understood that such embodiments are exemplary, and are not intended to limit the scope of the disclosure to those applications. For example, it will be understood that the devices, systems, and methods described herein are applicable to a variety of treatment procedures in which a balloon is used to occlude a body lumen or body cavity. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

As mentioned above, fluoroscopy-based approaches used in guiding intracavity therapy procedures, including navigation and deployment of a therapeutic balloon at the therapy site (e.g., blood vessel stenosis, pulmonary vein ostium), suffer from various drawbacks. It may be desirable to provide an approach for guiding a treatment procedure without using fluoroscopy and/or contrast agent. The present disclosure provides systems, methods, and devices for guiding a therapy procedure using electrical field detection for device visualization within an anatomical image or map. In particular, the present disclosure describes systems, methods, and devices for visualizing a reference location of a device relative to the current location of the device within the anatomical cavity.

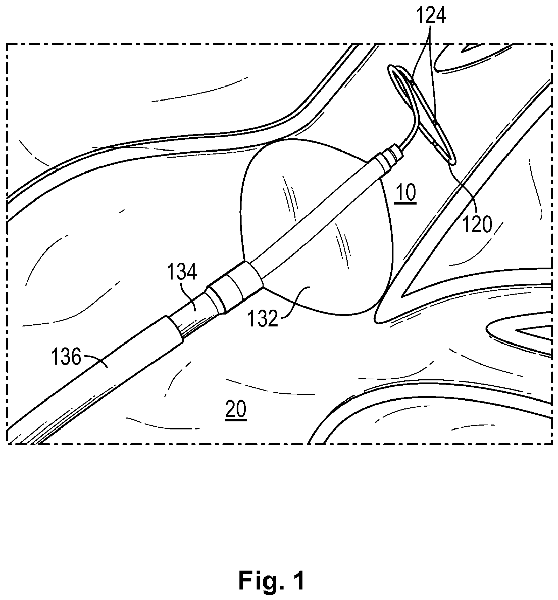

In some instances, imaging and/or therapeutic devices may unintentionally be moved from a desired location, such as a treatment site, during an imaging or therapeutic procedure. In that regard, Fig. 1 is a graphical view of an inflated ablation balloon 132 positioned at the ostium of the pulmonary vein 10. In an exemplary embodiment, the balloon 132 is cryoballoon. The cryoballoon 132 is configured to be inflated and then at least partially filled with a cooling fluid to cool the cryoballoon to a temperature (e.g., below -65° C) that causes an electrically-isolating lesion or firewall in the tissue. For example, the cryoballoon 132 may be in fluid communication with a source or reservoir of cooling fluid via one or more fluid lines positioned within a flexible elongate member 134. Further, the cryoballoon 132 may be in fluid communication with an air or gas source, e.g., for balloon inflation, via one or more fluid lines positioned within the flexible elongate member 134. The balloon 132 may be coupled to a distal portion of a flexible elongate member 134 that is protruding out a distal end of a sheath 136. In some embodiments, the sheath 136 is first introduced into the left atrium, and used to guide the EP catheter 120 to a reference location associated with an ablation site (e.g., pulmonary vein ostium). The balloon 132 may then be moved over the EP catheter 120 to the ablation site and positioned such that a full circumference of the balloon 132 is in contact with an entire circumference of the ostium of the pulmonary vein 10, and such that the ablation can be delivered around the entire circumference.

It is possible that, before moving the balloon 132 over the EP catheter 120 to the ablation site, the distal end of the EP catheter 120 carrying a plurality of electrodes 124 moves away from the desired location. For example, the balloon ablation procedure may involve one or more steps in which the balloon 132 is inflated, advanced toward from the PV ostium, or retracted away from the PV ostium. These movements of the balloon 132 are made relative to the EP catheter 120, which is positioned within a lumen of the sheath 136. Thus, in some instances, inflating and/or moving the balloon 132 may incidentally cause the position and/or orientation of the distal end of the EP catheter 120 relative to the anatomy to change. If the EP catheter 120 has substantially moved from its reference position, it is possible that the balloon 132 will not be correctly positioned to fully occlude the pulmonary vein 10 if the balloon 132 is guided over the EP catheter 120. Thus, the physician may choose to delay the ablation procedure until the EP catheter 120 can be repositioned and/or oriented at the reference location.

To ensure patient safety, it may be desirable to perform therapeutic procedures as quickly as possible. There is therefore a need to ensure that the physician can quickly guide an instrument, such as an imaging and/or therapeutic device, back to the reference location. Using the techniques described below, simultaneous visualization of the reference location and the current location allows the physician to more readily navigate the device back to the reference location if the device has moved. Accordingly, therapeutic workflows can be improved.

The foregoing may be achieved by a computer-implemented method configured to save (in a memory) parameters indicative of a reference location or treatment site (e.g., ablation site) for an imaging and/or treatment device. Such parameters may include (i) location, (ii) position, and (iii) orientation. Further, such parameters may be automatically determined or selected based on information set by a user, or manually selected by the user following an interaction with the user interface (e.g. clicking a button, saying a phrase, etc.). The saved parameters are translated into a visualization or graphical representation on a user interface relative to an anatomy of the heart in a manner that is distinctive (e.g. color, transparency, contrast) from a real time location of the same ablation catheter.

According to one embodiment of the present disclosure, a method for visualizing a reference location and current location of an instrument includes: receiving electrical signals from a plurality of electrodes disposed on the instrument; determining a plurality of parameters associated with a predetermined section of the instrument based on the electrical signals received by the plurality of electrodes, wherein said parameters are indicative of location, position and orientation of the predetermined section of the instrument; receiving, at a first time, a trigger or instruction to save the determined parameters of the predetermined section of the ablation device; displaying, on a display, the reference parameters relative to a representation of the instrument at the first time, and relative to the anatomical cavity; and displaying a real-time representation of the ablation device at a second time, which second time follows the first time, on the display concomitantly to the reference representation, wherein the reference representation differs from the real-time representation, thereby enabling the instrument to be aligned per the reference parameters at a third time.

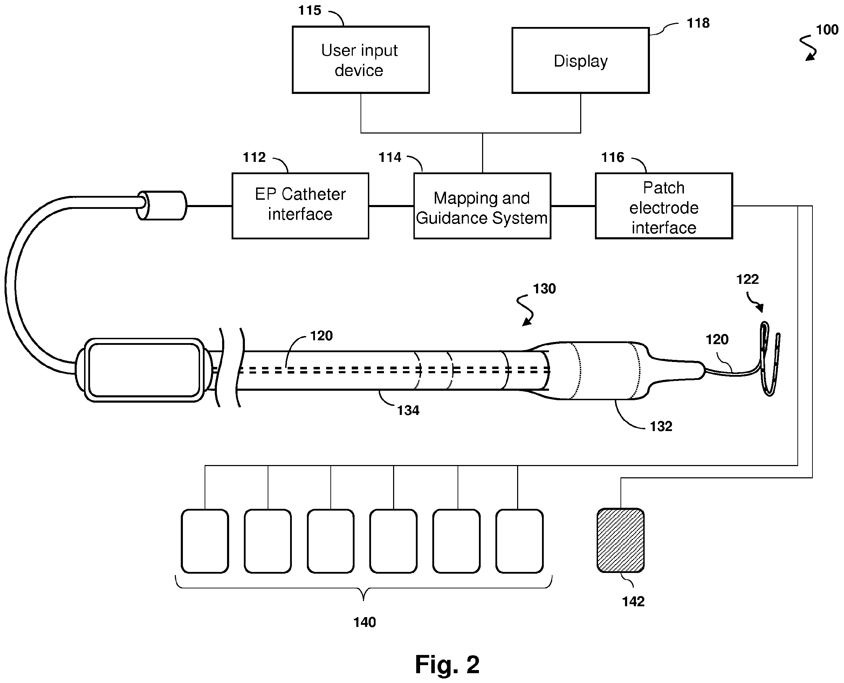

Fig. 2 is a diagrammatic schematic view of an EP cryoablation system 100, according to aspects of the present disclosure. The EP cryoablation system 100 includes an EP catheter 120. In some embodiments, the EP catheter 120 extends through a lumen of a cryoballoon catheter 130. The EP catheter 120 is communicatively coupled to an EP catheter interface 112, which is communicatively coupled to a mapping and guidance system 114. The cryoballoon catheter 130 may include an inflatable cryoballoon 132 coupled to a distal portion of a flexible elongate member 134, which may comprise a sheath. The EP catheter 120 may be positioned at least partially within a lumen of the flexible elongate member 134 such that a distal portion 122 of the EP catheter 120, which comprises a plurality of electrodes positioned on an elongate tip member, may protrude out a distal end of the cryoballoon catheter 130. For example, the cryoballoon catheter 130 may comprise a lumen configured to slidably receive the EP catheter 120. The EP catheter 120 may comprise a flexible elongate member configured to be positioned within the cryoballoon catheter 130. In some embodiments, the cryoballoon catheter 130 may be introduced into the body cavity first and the EP catheter 120 may be advanced distally within the cryoballoon catheter 130 until the distal portion 122 of the EP catheter 120 protrudes out of the distal end of the cryoballoon catheter 130 at a region of interest (e.g., an ablation site).

The distal portion 122 of the EP catheter may comprise a plurality of electrodes positioned on the elongate tip member. In some embodiments, the EP catheter comprises between 8 and 10 electrodes. However, the EP catheter can include other numbers of electrodes, including 2, 4, 6, 14, 20, 30, 60, or any other suitable number of electrodes, both larger and smaller. The elongate tip member may be configured to be positioned distally of the cryoballoon, and may be biased, shaped, or otherwise structurally configured to assume a shape, such as a circular shape, in which the electrodes are spaced from one another about one or more planes. For example, the EP catheter can be a spiral mapping catheter (SMC) in which electrodes are distributed along an elongate tip member in a spiral configuration. In some embodiments, commercially-available EP catheters can be used with the system 100, including the Achieve™ and Achieve Advance™ Mapping catheters manufactured by Medtronic™. The EP catheter can be designed for use with the Arctic Front™ Family of Cardiac Cryoablation Catheters and/or the FlexCath™ Advance Steerable Sheath, manufactured by Medtronic™. In some embodiments, the cryoballoon 132 comprises a plurality of electrodes positioned on an exterior surface of the cryoballoon 132 and configured to obtain data used to determine occlusion at an ablation site. Further details regarding EP catheters and assemblies can be found in, for example, U.S. Patent No. 6,002,955, titled "Stabilized Electrophysiology Catheter and Method for Use," the entirety of which is hereby incorporated by reference.

The system 100 further comprises a plurality of body patch electrodes 140 and a reference patch electrode 142 communicatively coupled to a patch electrode interface 116, which is in communication with the mapping and guidance system 114. For example, the patch electrodes 140 and the reference electrode 142 may be coupled to the patch electrode interface 116 via electrical cables. In the embodiment shown in Fig. 2, the system 100 comprises six external body patch electrodes 140 and one reference electrode 142. However, in some embodiments, the system 100 comprises fewer or more body patch electrodes 140 than are shown in Fig. 2, including 1, 2, 4, 8, 10, 12, 20, or any other suitable number of body patch electrodes, both lager and smaller. Further, in some embodiments, the system 100 may include more than one reference electrode 142, including 2, 4, 5, or any other suitable number of reference patch electrodes.

The patch electrodes 140 and reference electrode 142 may be used to generate images or models of a body cavity, such as the chambers of the heart, body lumens that provide access to the body cavity, and other features identified to be electrically isolated from the body cavity (e.g., the pulmonary vein). For example, the patch electrodes 140 may be used in pairs to generate electrical fields in different directions and at different frequencies within a body cavity of a patient. The patch electrodes 140 may be controlled by the mapping and guidance system 114 and/or the patch electrode interface 116 to generate the electrical fields. Mapping data is obtained by the electrodes of the EP catheter 120 by detecting distortions in the electrical fields. The mapping data may then be used to generate the image or model of the body volume, such as a cavity of the heart. The mapping and guidance system 114 may then output the generated map of the cavity to a display.

Further, the mapping and guidance system 114 may be configured to determine a location of the EP catheter within the body cavity and output a visualization to the display that indicates a position of the EP catheter 120 within the map. For example, based on the mapping data acquired by the electrodes of the EP catheter, the mapping and guidance system 114 may be configured to determine the position of the electrodes of the EP catheter, and output a visualization indicating the position of each of the electrodes on the map. Further, based on the determined position of the electrodes of the EP catheter, the mapping and guidance system 114 may be configured to determine or estimate a location of the cryoballoon 132 within the body cavity. A more detailed explanation of the use of body patch electrodes and catheter electrodes to map body volumes and visualize the locations of EP catheters within the map can be found in, for example, U.S. Patent No. 10,278,616, titled "Systems and Methods for Tracking an Intrabody Catheter," filed May 12, 2015, and U.S. Patent No. 5,983,126, titled "Catheter Location System and Method," filed August 1, 1997, the entireties of which are hereby incorporated by reference.

In the diagram shown in Fig. 2, the EP catheter interface 112, mapping and guidance system 114, and patch electrode interface 116 are illustrated as separate components. However, in some embodiments, the interfaces 112, 116 and the mapping and guidance system 114 may be components of a single console or computing device with a single housing. In other embodiments, the interfaces 112, 116 and the mapping and guidance system 114 may comprise separate hardware components (e.g., with separate housings) communicatively coupled to one another by electrical cables, wireless communication devices, fiber optics, or any other suitable means of communication. Further, in some embodiments, the EP catheter interface 112 may also function as an interface for the cryoballoon catheter 130 to control inflation of the cryoballoon 132, cooling/heating of the cryoballoon 132, etc. In other embodiments, the cryoballoon catheter 130 is controlled by a separate interface or control system, such as a console.

The mapping and guidance system 114 is coupled to a display device 118, which may be configured to provide visualizations of a cryoablation procedure to a physician. For example, the mapping and guidance system 114 may be configured to generate EP images of the body cavity, visualizations of the propagation of EP waves across the tissue of the body cavity, indications of occlusion by the cryoballoon at an ablation site, or any other suitable visualization. These visualizations may then be output by the mapping and guidance system 114 to the display device 118.

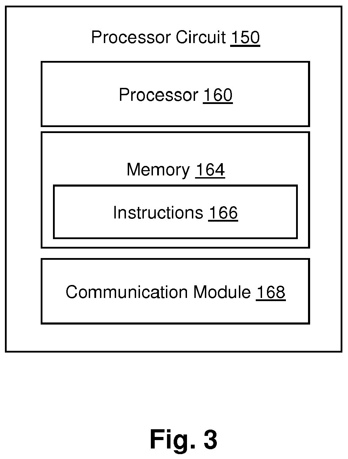

Fig. 3 is a schematic diagram of a processor circuit 150, according to embodiments of the present disclosure. The processor circuit 150 may be implemented in the mapping and guidance system 114, the EP catheter interface 112, the patch electrode interface 116, and/or the display device 118. The processor circuit 150 can carry out one or more steps described herein. As shown, the processor circuit 150 may include a processor 160, a memory 164, and a communication module 168. These elements may be in direct or indirect communication with each other, for example via one or more buses.

The processor 160 may include a central processing unit (CPU), a digital signal processor (DSP), an ASIC, a controller, an FPGA, another hardware device, a firmware device, or any combination thereof configured to perform the operations described herein. The processor 160 may also be implemented as a combination of computing devices, e.g., a combination of a DSP and a microprocessor, a plurality of microprocessors, one or more microprocessors in conjunction with a DSP core, or any other such configuration.

The memory 164 may include a cache memory (e.g., a cache memory of the processor 160), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and nonvolatile memory, or a combination of different types of memory. In an embodiment, the memory 164 includes a non-transitory computer-readable medium. The non-transitory computer-readable medium may store instructions. For example, the memory 164, or non-transitory computer-readable medium may have program code recorded thereon, the program code including instructions for causing the processor circuit 150, or one or more components of the processor circuit 150, to perform the operations described herein. For example, the processor circuit 150 can execute operations of the methods 200 and 500. Instructions 166 may also be referred to as code or program code. The terms "instructions" and "code" should be interpreted broadly to include any type of computer-readable statement(s). For example, the terms "instructions" and "code" may refer to one or more programs, routines, subroutines, functions, procedures, etc. "Instructions" and "code" may include a single computer-readable statement or many computer-readable statements. The memory 164, with the code recorded thereon, may be referred to as a computer program product.

The communication module 168 can include any electronic circuitry and/or logic circuitry to facilitate direct or indirect communication of data between the processor circuit 150, the mapping and guidance system 114, the EP catheter 120, the cryoballoon catheter 130, and/or the display 118. In that regard, the communication module 168 can be an input/output (I/O) device. In some instances, the communication module 168 facilitates direct or indirect communication between various elements of the processor circuit 150 and/or the system 100 (Fig. 2). In some embodiments, the processor circuit 150 may further comprise an electrical signal generator and/or an electrical signal measurer configured to control the electrodes of the EP catheter to emit and/or detect electrical signals, including voltages, impedances, and currents.

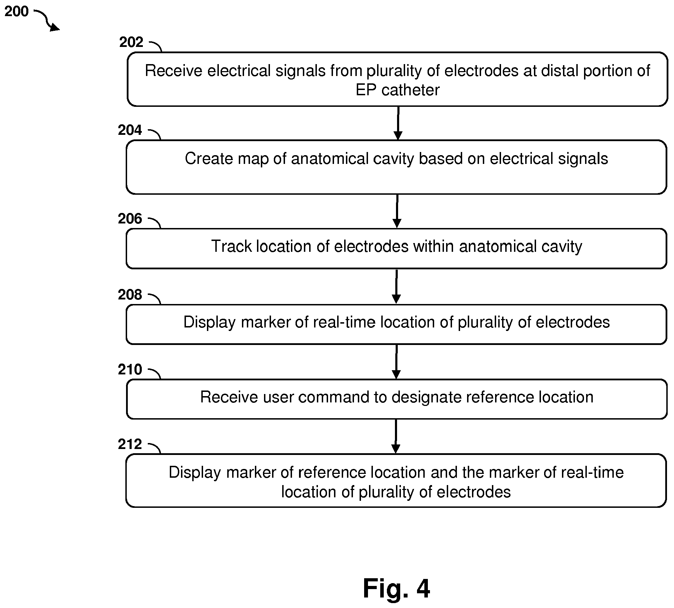

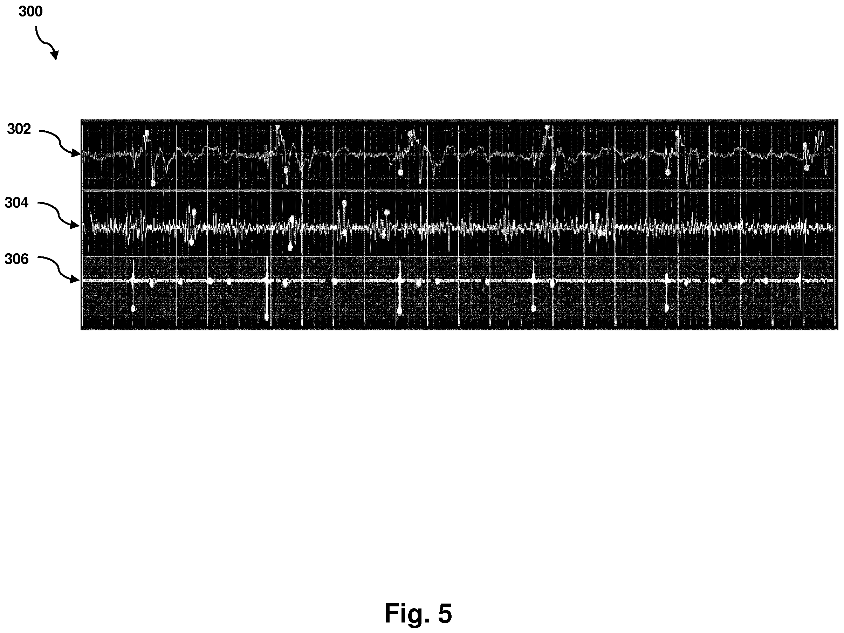

Fig. 4 is a flowchart illustrating a method 200 for visualizing a reference location of an instrument for electrophysiological mapping and therapy, according to some embodiments of the present disclosure. Figs. 5-8 illustrate steps and aspects of the method 200. Referring to Fig. 4, it will be understood that some or all of the steps of the method 200 may be performed using one or more components of the system 100 shown in Fig. 2, including the EP catheter 120, the cryoballoon catheter 130, the mapping and guidance system 114, and/or the patch electrodes 140, 142. In step 202, a processor circuit receives electrical signals from a plurality of electrodes positioned within an anatomical cavity. In some embodiments, the electrical signals are representative of electrical measurements (voltage, conductance, etc.) of the anatomical cavity. The electrical measurements may be representative of an electrical field induced in the anatomical cavity by external body patch electrodes and/or by the plurality of electrodes within the anatomical cavity. Fig. 5 shows a graph 300 of a plurality of electrical signals or electrograms 302, 304, 306 obtained by the plurality of electrodes. The electrical signals 302, 304, 306 are measured as a voltage, and may be quantified in millivolts (mV). In some embodiments, the processor circuit is configured to output, to a display, a user interface that includes visualizations of the electrograms 302, 304. 306.

In an exemplary embodiment, the plurality of electrodes is positioned at a distal tip or portion of a flexible elongate member, such as a catheter. For example, in some embodiments, an electrophysiology (EP) catheter carrying the plurality of electrodes is navigated to an anatomical cavity, such as a chamber of the heart or a diseased blood vessel. Navigating the EP catheter to the anatomical cavity is performed using EP imaging techniques to image the full anatomical cavity. For example, in some embodiments, a guidewire with electrodes, a sheath with electrodes, and/or the EP catheter is inserted into an entry site, such as the femoral vein in the patient's leg and navigated to the anatomical cavity (e.g., left atrium) while the access route is imaged to guide the placement of the guidewire, sheath, or catheter at the right atrium. In some embodiments, the EP catheter is introduced into the left atrium using a transseptal procedure. In some embodiments, the transseptal procedure may involve the use of a transseptal needle or RF needle to penetrate the transseptal wall to introduce the catheter or sheath into the left atrium. The EP catheter is moved around within the anatomical cavity, obtaining electrical measurements (e.g., voltage, conductance) at various locations within the cavity.

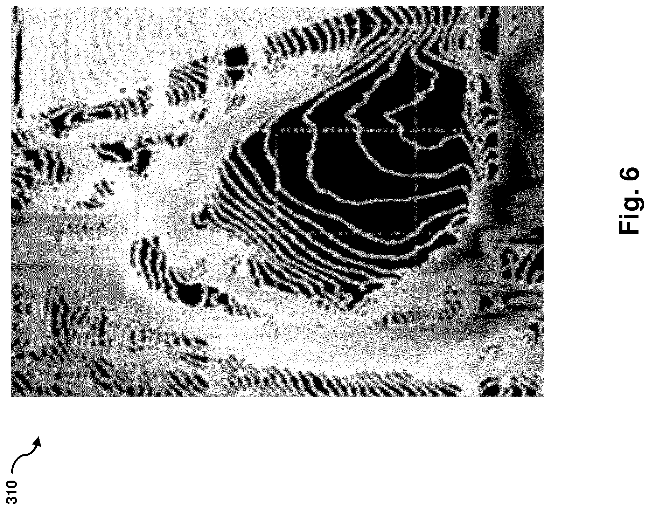

In some embodiments, multiple body patch electrodes are utilized to induce electrical fields through the anatomy of the patient. These electrical fields are measured by the electrodes obtaining electrical measurements or electrograms. The anatomy of the patient causes distortions in the induced electrical fields, resulting in non-homogenous electric fields. The bending of the electric fields caused by the anatomy can be detected by measuring the voltages at each of the catheter electrodes. In particular, each catheter electrode measures the potential (voltage) associated with each of the electric fields induced by the body patch electrodes. In some embodiments, different pairs or subsets of body patch electrodes induce different electric fields (*e.g.,* different frequencies). The processor circuit detects the changes in the electric fields caused by the anatomy based on the voltage measurements at the catheter electrodes. In that regard, Fig. 6 illustrates a topographical view 310 of a non-homogenous electrical field within a chamber of the heart, such as the left atrium. The various tissue structures surrounding the left atrium cause distortions to the electrical field induced by body patch electrodes. The spatial variances and/or distortions of the electrical field can be detected by the plurality of electrodes. In particular, the processor circuit converts the voltage measurements (mV) of the catheter electrodes into corresponding distances (mm) based on preconfigured calibration data associated with the particular catheter currently in use.

In step 204, the processor circuit generates a map or image of the anatomical cavity based on the electrical signals. In particular, the detected distortions in the electrical field are used to locate tissue structures surrounding an anatomical cavity. In some embodiments, preconfigured calibration data and/or other information associated with the electrodes is stored on a memory in communication with the processing system. The physician may select an instrument (e.g., EP catheter) being used from a dropdown menu to identify and recall the associated preconfigured calibration data for that particular instrument. Using the preconfigured calibration data for the instrument carrying the plurality of electrodes, and known spatial relationships of the electrodes (e.g., spacing, order), the processor circuit solves equations representing the positions of the catheter electrodes in such a manner that at least a threshold percentage (e.g., 80%, 90%, 95%) of all locations of the electrodes match an allowed orientation of the electrodes as determined by the calibration process. Based on the bending of the electric fields (*e.g*., gradient) as measured by the voltages at the electrodes and corresponding positions of the electrodes, the anatomical image is created.

In some embodiments, the plurality of electrodes positioned within the anatomical cavity are also configured to emit an electrical signal at a particular frequency. Further, all of the electrodes may be configured to detect the electrical signals emitted by the other electrodes at the other frequencies, in addition to the electrical signal emitted by the electrode. For example, a first electrode may be configured to emit a first electrical signal at a first frequency, and a second electrode may be configured to emit a second electrical signal at a second frequency. In an exemplary embodiment, the first electrode is used to detect the first electrical signal at the first frequency, and the second electrical signal is used to detect the second electrical signal at the second frequency. However, in other embodiments, the first electrode may detect the second electrical signal at the second frequency in addition to its own first electrical signal at the first frequency. Similarly, the second electrode may detect the first electrical signal at the first frequency in addition to its own second electrical signal at the second frequency.

The plurality of electrodes is moved around the anatomical cavity to obtain electrical field measurements at a variety of locations. Using the information obtained by the plurality of electrodes moving to a plurality of locations within the cavity, a voltage field map of the electromagnet field can be generated. Based on the voltage map, the distortions in the electrical field can be computed by the processor circuit. The processor circuit uses these detected distortions to locate the tissue structures and build a map.

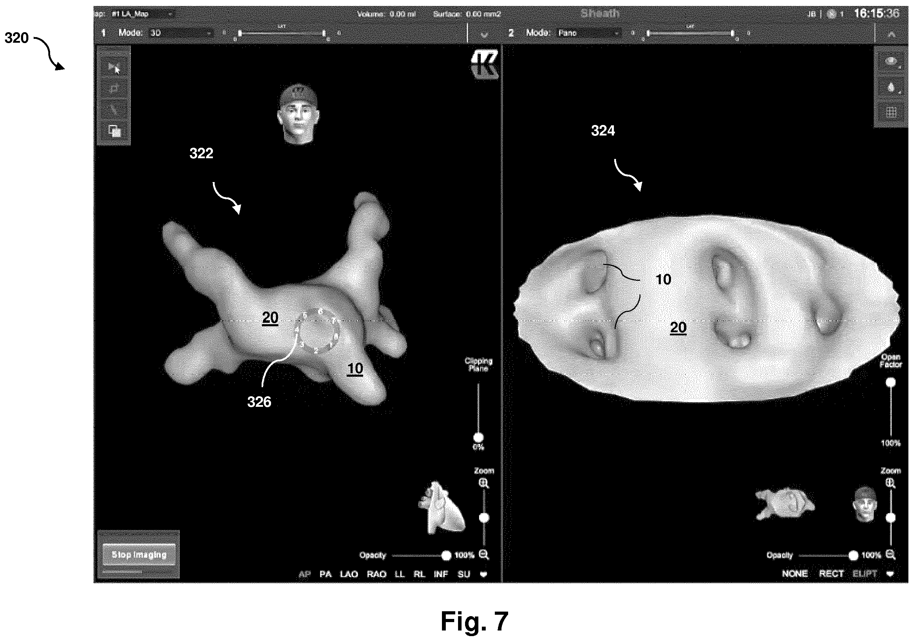

With reference to step 204 of the method 200, Fig. 7 shows a user interface 320 that includes three-dimensional anatomical images 322, 324, or views of the left atrium. For example, a first atrial view 322 is a perspective three-dimensional view of the internal structure of the left atrium 20 and pulmonary veins 10. The view 322 includes an EP catheter indicator 326 that shows a location and/or orientation of the electrodes at the distal portion or an EP catheter. In some embodiments, the view 322 can be manipulated (e.g., oriented, resized, moved) by a user to provide different views or angles of the left atrium. A second atrial view 324 may comprise a flattened panoramic view of the left atrium 20. For example, the second view 324 may be generated by translating, stretching, distorting, or otherwise modifying the first view 322. The second view 324 shows the ostia of the pulmonary veins in a 3D flattened configuration, which may be advantageous for planning and performing ablation procedures, particularly in order to carry out the full ablation procedure without repeated manipulation and frequent changes in projection of the 3D reconstructed image. The views 322, 324 can be used to locate the desired treatment site, and to guide placement of a therapeutic balloon at the treatment sites, and/or otherwise plan the treatment procedure. In some embodiments, one or more locations of the EP catheter are recorded during the mapping procedure described with respect to step 204.

In step 206, the processor circuit tracks the location of the plurality of electrodes based on the detected electrical field. In an exemplary embodiment, the processor circuit determines the location based on detected distortions in the electrical field(s) within the anatomical cavity. In one embodiment, the plurality of electrodes is positioned on a distal tip or portion of an EP catheter. The distal tip of the EP catheter may be shaped in a circle, ellipse, loop, or biased to maintain such shapes, such that the electrodes are spaced from one another in various directions in three-dimensional space. In some embodiments, the electrodes are configured to continuously or repeatedly measure the electrical field and transmit electrograms or electrical signals representative of the electrical field to the processor circuit. In some embodiments, the electrodes used to emit the EM field include a plurality of body patch electrodes, a plurality of catheter-mounted electrodes, and/or any other suitable type of electrode. Further, it will be understood that the EM field may include multiple EM fields having different parameters or properties, such as different frequencies. In an exemplary embodiment, the electrodes of the EP catheter 120 shown in Fig. 2 may be used to emit the EM field. The processor circuit detects or computes distortions in the electrical field, and determines a current or real-time location of the electrodes based on the detected distortions.

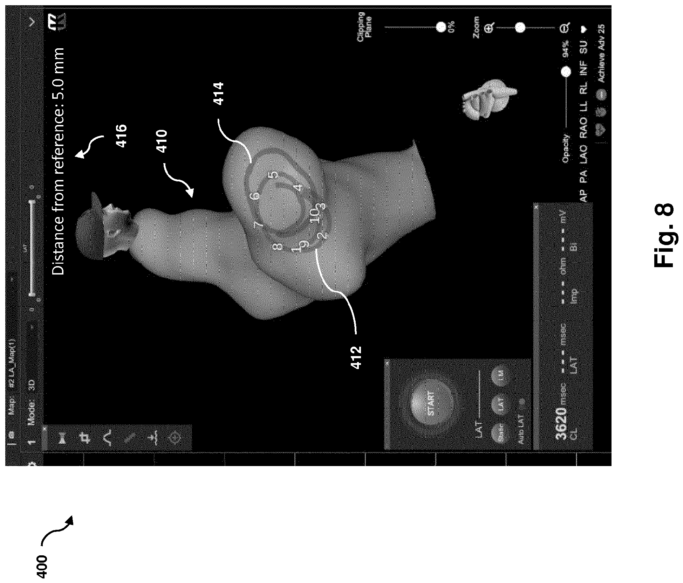

In step 208, the processor circuit displays a marker of the real-time location of the plurality of electrodes. In some aspects, displaying the marker may include generating a signal representative of the marker based on the determined location, and outputting the signal to a display in communication with the processor circuit. The signal may comprise any suitable type of communication, such as an electrical signal (e.g., digital electrical signal) representative of image data. For example, the electrical signal may be in a format suitable for display by a display device (e.g., computer monitor, television screen, mobile computing device display, etc.). In some embodiments, step 208 includes displaying the marker with respect to a view or map of the anatomical cavity as generated in step 204. For example, the marker may be overlaid on the anatomical map generated as described above. In that regard, Fig. 8 depicts a graphical user interface 400 that includes a three-dimensional anatomical image 410 of a left atrium, and a marker 412 of the current location of the EP catheter. The marker 412 comprises a shape that is representative of the shape of the distal portion of the EP catheter on which the electrodes are disposed. The marker 412 is output to the display at a position relative to the anatomical image 410 to illustrate the current location of the distal portion of the EP catheter within the anatomy, as determined in step 206. Accordingly, the position, shape, and orientation of the marker 412 illustrates the position, shape, and orientation of the distal portion of the EP catheter, in three-dimensions. Further, the marker 412 includes electrode indicia that show the arrangement and relative positions of the individual electrodes at the distal portion of the EP catheter. In particular, the electrode indicia include numerals identifying the corresponding electrode. Because the shape of the distal portion of the EP catheter may change during the procedure, the processor circuit may be configured to update the shape of the marker 412 to show the changes in the shape, orientation, and/or location of the distal portion of the EP catheter.

In step 210, the processor circuit receives a user input or command to designate a particular instantaneous location of the plurality of electrodes as a reference location. In some embodiments, the user input may be received from a user input device, such as keyboard, computer mouse, touch screen display, physical button, microphone, foot pedal, or any other suitable type of user input device. For example, once the physician has determined, by viewing the marker 412 of the current location of the EP catheter, that the distal portion of the EP catheter is positioned at a desired reference location, the physician may trigger the designation of the location as the reference location by pressing a key on a keyboard, clicking a mouse button, or speaking a phrase into a microphone of a voice command system. The processor circuit then designates the location as the reference location by, for example, saving the electrical parameters or measurements associated with the location to a memory.

In step 212, the processor circuit displays a second marker of the reference location simultaneously with the marker of the current, or real-time location of the plurality of electrodes. For example, referring again to Fig. 8, a second marker 414 of the reference location designated in step 210 is shown relative to the first marker 412. The second marker 414 of the reference location may be referred to as a landmark or a shadow for the purposes of the present disclosure. Thus, when the current location of the EP catheter deviates from the reference location and/or orientation, the physician can determine the relative position, orientation, and/or extent of deviation in order to navigate the EP catheter back to the reference location and orientation.

In Fig. 8, the first marker 412 of the current location and the second marker 414 of the reference location are created and generated in a same procedure. For example, a physician may trigger the designation of the reference location to display the second marker 414 while the first marker 412 continues to be displayed. Alternatively, in some embodiments, the parameters associated with the second marker 414 may be recalled in a later procedure. In that regard, the reference location may be designated in an imaging procedure at a first time, before performing a therapy. The imaging procedure may be paused or terminated before returning to perform the therapeutic procedure at a later second time. During the later therapeutic procedure, the first marker 412 is again shown within the anatomical cavity of the anatomical image. The parameters associated with the reference location are recalled and the second marker 414 is displayed simultaneously with the first marker 412. Accordingly, the physician is allowed more flexibility in determining how and when to perform the imaging and therapy procedures. In that regard, it will be understood that, while the visualization techniques described herein may refer to visualizing current and reference locations of electrodes as part of a therapy procedure, the present disclosure contemplates that the visualization techniques may be performed during an imaging procedure that does not include therapy. Further, the present disclosure also contemplates the visualization of the current and reference locations in which both imaging and therapy procedures are performed.

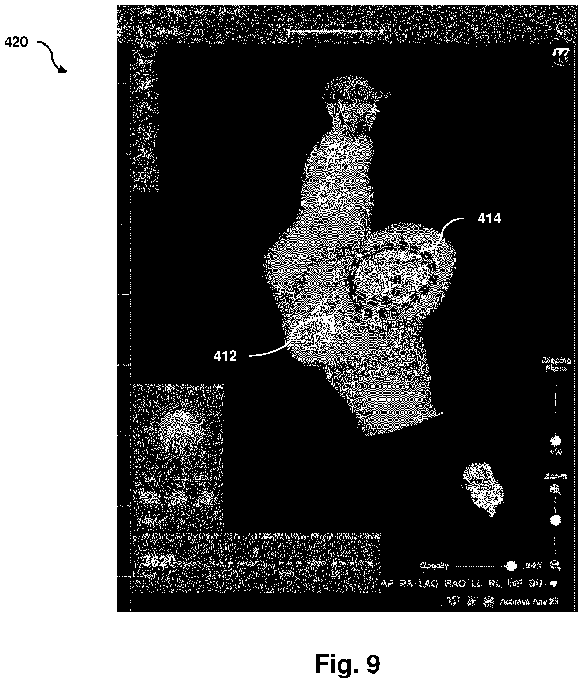

In some embodiments, the processor circuit is further configured to calculate a distance between the reference location and the current location of the plurality of electrodes, and output a graphical representation of the distance to the display. In that regard, Fig. 8 shows a textual indicator 416 that provides a real-time distance measurement between the current location and the reference location. The second marker 414 may have one or more visual characteristics that differ from one or more corresponding visual characteristics of the first marker 412. For example, the second marker 414 may have a different color, degree of transparency, brightness, pattern, outline, or any other suitable visual characteristic that can indicate the distinction between the first marker 412 and the second marker 414. As shown in Fig. 8, only the first marker 412, and not the second marker 414, includes the electrode indicia. Fig. 9 illustrates an alternative embodiment of a graphical user interface 420 in which the reference location marker 414, which may be referred to as a landmark, is shown with a dashed line pattern, while the first marker 412 of the current location is a solid line having electrode indicia.

In some embodiments, the reference location is not determined based on a trigger signal provided by a user, but by pre-defined parameters saved to memory. For example, in some embodiments, the processor circuit is configured to analyze the anatomical image to identify an anatomical feature of interest. The anatomical feature of interest may be associated with a treatment location and/or a reference location. For example, the anatomical feature may be a pulmonary vein or pulmonary vein ostium. Once the anatomical feature of interest is identified, the processor circuit is configured to determine a desired reference location automatically. In some embodiments, the user input is representative of a location within the anatomical image. For example, instead of triggering a save-to-memory of the currently displayed location of the electrodes, the physician may select, using a touch screen interface, a trackball, mouse, or other user interface device, a location on the anatomical image. The processor circuit may then output the reference marker based on the user input.

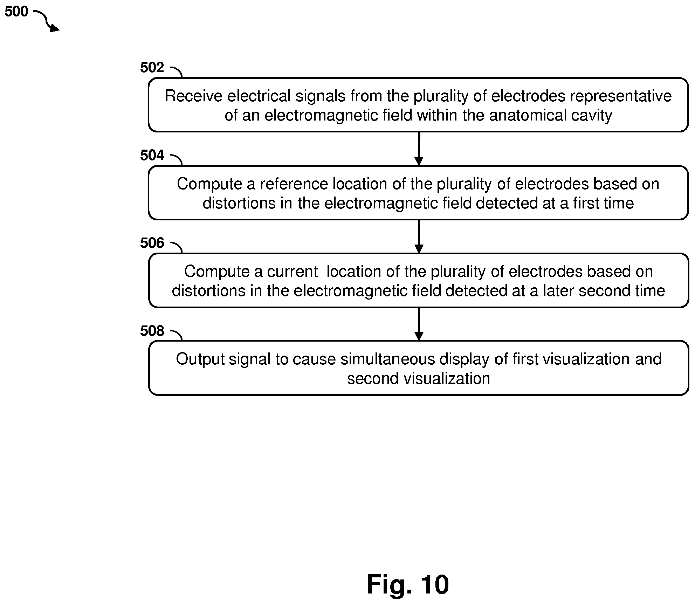

Fig. 10 is a flowchart illustrating a method 500 for visualizing a reference location of an instrument for electrophysiological mapping and therapy, according to some embodiments of the present disclosure. It will be understood that some or all of the steps of the method 500 may be performed using one or more components of the system 100 shown in Fig. 2, including the EP catheter 120, the cryoballoon catheter 130, the mapping and guidance system 114, and/or the patch electrodes 140, 142.

In step 502, the processor circuit receives electrical signals from the plurality of electrodes representative of an electromagnetic field within the anatomical cavity. In some embodiments, the processor circuit is configured to generate an anatomical image or map based on detected distortions in the electromagnetic field as described above. The anatomical image may be output to a display so that it can be used in the other steps of the method 500.

In step 504, the processor circuit computes a reference location of the plurality of electrodes based on distortions in the electromagnetic field detected at a first time. The processor circuit determines the distortions based on the received electrical signals, or electrograms. Based on the determined distortions, the processor circuit computes the reference location. In some embodiments, computing the reference location includes comparing electrical parameters associated with the electrical signals to parameters previously recorded when generating the anatomical image. In some embodiments, computing the reference location comprises designating or saving a location of the plurality of electrodes in response to a user input, instruction, or command to designate and/or save the location as the reference location. In some embodiments, the processor circuit receives the user input from a user input device such as a keyboard, mouse, button, trackpad, touch screen interface, microphone, or any other suitable type of user input device.

In step 506, the processor circuit computes a current location of the plurality of electrodes based on distortions in the electromagnetic field detected at a later second time. In some embodiments, the processor circuit receives a continuous stream of electrical signals, and continuously or repeatedly computes the current location of the plurality of electrodes to track the position and/or orientation of the plurality of electrodes within the anatomical cavity.

In step 508, the processor circuit outputs a signal configured to cause simultaneous display of a first visualization of the reference location and a second visualization of the current location such that the current location is displayed relative to the reference location. The signal may comprise any suitable type of communication, such as an electrical signal (e.g., digital electrical signal) representative of image data. For example, the electrical signal may be in a format suitable for display by a display device (e.g., computer monitor, television screen, mobile computing device display, etc.). In an exemplary embodiment, the processor circuit outputs the first and second visualizations such that a real-time view of the current location of the plurality of electrodes is shown relative to the previously determined reference location. In other words, the first visualization may be repeatedly or continuously updated based on the electrical signals to show the current, real-time location of the plurality of electrodes. In this way, when the current location/orientation of the electrodes deviates from the reference location/orientation, the physician can determine the relative location, orientation, and/or extent of deviation in order to navigate the electrodes back to the reference location and orientation.

In some embodiments, the first visualization has a visual characteristic that is different from a visual characteristic of the second visualization. The visual characteristic may be a color, transparency, electrode indicia, pattern, or any other suitable visual characteristic. For example, the electrode indicia may be present on the second visualization of the current location of the electrodes, but not the first visualization. The electrode indicia may include numerical representations associated with each of the plurality of electrodes. In some embodiments, the processor circuit is further configured to calculate a distance between the reference location and the current location and output the signal to cause simultaneous display of the first visualization, the second visualization, and a third visualization of the calculated distance. Further, in some embodiments, the processor circuit is configured to generate and output a visualization of a position of a therapeutic tool, such as an ablation balloon. Further details related to generating and outputting visualizations of therapeutic tools can be found in European Patent Application No. 19206883.1, filed November 4, 2019, titled "ELECTRPHYSIOLOGICAL GUIDANCE AND VISUALIZATION FOR BALLOON THERAPY AND ASSOCIATED DEVICES, SYSTEMS, AND METHODS," the entirety of which is hereby incorporated by reference.

The embodiments of the present disclosure provide for image-guided therapy by detecting and visualizing a location of a plurality of electrodes, such as electrodes positioned on an EP catheter, an ablation catheter, or a guidewire, within an anatomical cavity of a patient. However, it will be understood that the embodiments described above are exemplary and are not intended to limit the scope of the present disclosure. In that regard, in some embodiments, the approaches of the methods 200 and 500 described above may be to detect and/or visualize other types of therapeutic and/or diagnostic devices to guide other types of therapies, such as balloon angioplasty, laser balloon therapy, balloon breast brachytherapy, drug delivery using drug-coated balloons, neuro-radiology, etc.

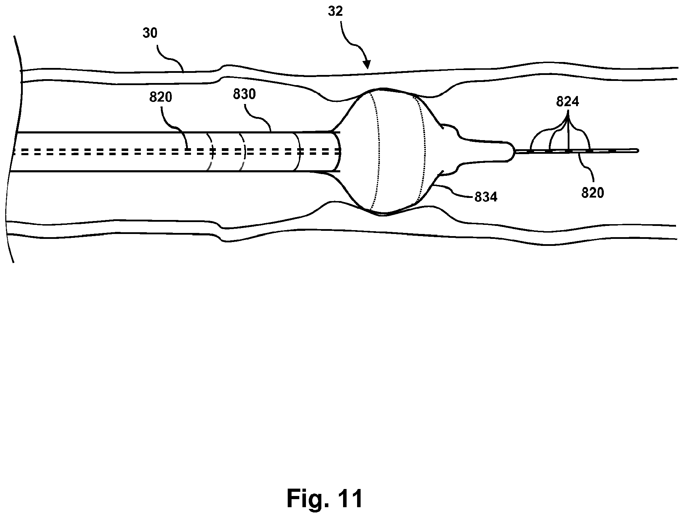

For example, with reference to Fig. 11, a guidewire 820 having a plurality of electrodes 824 at a distal end of the guidewire 820 can be advanced into a stenosed section 32 of a blood vessel 30 to obtain EP data and/or anatomical mapping data of the vessel 30. Accordingly, an anatomical map of the vessel 30 may be generated and displayed. It will be understood that the anatomical map of the vessel 30 may appear similar to the maps generated for other parts of the anatomy, such as the map of the left atrium and pulmonary veins shown in Fig. 7. Further, similar to the embodiments shown in Figs. 8 and 9, the reference location and the current location of the guidewire electrodes 824 can be computed and displayed within an anatomical image of the vessel 30. For example, markers corresponding to the current location and reference location can be shown using the techniques of the methods 200 and 500 described above.

The anatomical map of the vessel 30 may show a cross-sectional view, a partially-transparent view, a volumetric view, or any other suitable view. In some embodiments, the processor circuit is further configured to visualize a location of the guidewire 820 within the map of the vessel 30. Next, an angioplasty balloon catheter 830 is advanced over the guidewire 820 to the stenosed region 32. The angioplasty balloon 834 is then inflated to apply expand or dilate the stenosed region 32.

In still other embodiments, a drug-coated balloon, a radiation balloon, a scoring balloon, or any other suitable treatment balloon is advanced over a guidewire or catheter to a treatment site. Using one or more of the approaches described above, the therapy balloon can be spatially localized and visualized within the vessel relative to the treatment site (e.g., stenosis, lesion, tumor, etc.) to ensure proper placement and deployment of the therapy balloon. This approach may be particularly advantageous in neuro radiology and/or vascular balloon therapy procedures in which the therapeutic balloons are too small to carry electrodes for visualization.

It will be understood that one or more of the steps of the methods 200 and 500 can be performed by one or more components of an EP-guided ablation system, such as a processor circuit of a mapping and guidance system, an EP catheter, a cryoballoon catheter, an RF ablation balloon catheter, external body patch electrodes, or any other suitable component of the system. For example, the described ablation procedures may be carried out by the system 100 described with respect to Fig. 2, which may include the processor circuit 150 described with respect to Fig. 3. In some embodiments, the processor circuit can use hardware, software or a combination of the two to perform the analyses and operations described above. For example, the result of the signal processing steps of the methods 200 and 500 may be processed by the processor circuit executing a software to make determinations about the locations of the electrode in the 3D image, etc.

It will also be understood that the embodiments described above are exemplary and are not intended to limit the scope of the disclosure to a given clinical application. For example, as mentioned above, the devices, systems, and techniques described above can be used in a variety of ablation applications that involve occlusion of a body cavity or body lumen. For example, in some embodiments, the techniques described above can be used to guide a cryoablation procedure using a cryocatheter comprising a cryoballoon as described above. In other aspects, the techniques described above can be used to guide an RF ablation procedure in which a plurality of RF ablation electrodes positioned on the surface of an inflatable balloon are used to create an electrically-isolating lesion in cardiac tissue. For example, the HELIOSTAR RF balloon catheter, manufactured by Biosense Webster, Inc., includes 10 ablation electrodes positioned on an external surface of the inflatable balloon, and 10 electrodes on a circular mapping catheter positioned distally of the balloon and configured to be positioned inside the pulmonary vein.

Further, while the imaging and therapeutic procedures are described with respect to the heart and associated anatomy, it will be understood that the same methods and systems can be used to guide imaging and therapeutic procedures in other body volumes, including other regions of interest in the heart, or other body cavities and/or lumens. For example, in some embodiments, the procedures described herein can be used to guide treatment procedures in any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers or other parts of the heart, and/or other systems of the body. The anatomy may be a blood vessel, as an artery or a vein of a patient's vascular system, including cardiac vasculature, peripheral vasculature, neural vasculature, renal vasculature, and/or any other suitable lumen inside the body. In addition to natural structures, the approaches described herein may be used to examine man-made structures such as, but without limitation, heart valves, stents, shunts, filters and other devices. in the kidneys, lungs, or any other suitable body volume.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. An apparatus for guiding an instrument within an anatomical cavity, comprising:
a processor circuit configured for communication with a plurality of electrodes disposed on the instrument, wherein the processor circuit is configured to:
r receive electrical signals from the plurality of electrodes representative of an electromagnetic field within the anatomical cavity;
compute a reference location of the plurality of electrodes based on distortions in the electromagnetic field detected at a first time;
compute a current location of the plurality of electrodes based on distortions in the electromagnetic field detected at a later second time; and
output a signal configured to cause simultaneous display of a first visualization of the reference location and a second visualization of the current location such that the current location is displayed relative to the reference location.

2. The apparatus of claim 1, wherein a visual characteristic of the first visualization is different from the visual characteristic of the second visualization.

3. The apparatus of claim 2, wherein the visual characteristic comprises at least one of: a color, a transparency, an electrode indicium, or a pattern.

4. The apparatus of claim 3, wherein the second visualization, and not the first visualization, includes electrode indicia representative of relative positions of the plurality of electrodes.

5. The apparatus of claim 4, wherein the electrode indicia include numerical representations associated with each electrode of the plurality of electrodes.

6. The apparatus of claim 1, wherein the processor circuit is configured to:
receive a user input indicating an instruction to compute the reference location; and
compute the reference location in response to receiving the user input.

7. The apparatus of claim 6, wherein the processor circuit is configured to save parameters representative of the reference location to a memory in response to receiving the user input.

8. The apparatus of claim 1,
wherein the processor circuit is further configured to generate, based on the electrical signals from the plurality of electrodes, an anatomical image of the anatomical cavity, and
wherein the processor circuit is configured to output the signal such that the first visualization is stationary within the anatomical image.

9. The apparatus of claim 1, wherein the processor circuit is further configured to:
calculate a distance between the reference location and the current location of the plurality of electrodes; and
output the signal to cause simultaneous display of the first visualization, the second visualization, and a third visualization of the calculated distance.

10. The apparatus of claim 1, wherein the processor circuit is configured to:
repeatedly compute the current location of the plurality of electrodes; and
repeatedly update the second visualization to display the current location of the plurality of electrodes.

11. The apparatus of claim 1, wherein the processor circuit is configured to output the signal to a display in communication with the processor circuit.

12. A system for guiding an instrument within an anatomical cavity, comprising:
the apparatus as in any of claims 1-11; and
the instrument, wherein the instrument comprises an electrophysiology (EP) catheter comprising an elongate tip member, wherein the plurality of electrodes is positioned on the elongate tip member,
wherein the first visualization is representative of the elongate tip member of the EP catheter at the first time, and
wherein the second visualization is representative of the elongate tip member of the EP catheter at the later second time.

13. A method for guiding an instrument within an anatomical cavity, comprising:
receiving electrical signals from a plurality of electrodes disposed on the instrument, wherein the electrical signals are representative of an electromagnetic field within the anatomical cavity;
computing a reference location of the plurality of electrodes based on distortions in the electromagnetic field detected at a first time;
computing a current location of the plurality of electrodes based on distortions in the electromagnetic field detected at a later second time; and
outputting a signal configured to cause simultaneous display of a first visualization of the reference location and a second visualization of the current location such that the current location is displayed relative to the reference location.

14. The method of claim 13, further comprising:
receiving a user input indicating an instruction to compute the reference location; and
computing the reference location in response to receiving the user input.

15. A computer program product comprising:
a non-transitory computer-readable medium having program code recorded thereon, the program code including:
code for receiving electrical signals from a plurality of electrodes disposed on an instrument, wherein the electrical signals are representative of an electromagnetic field within an anatomical cavity;
code for computing a reference location of the plurality of electrodes based on distortions in the electromagnetic field detected at a first time;
code for computing a current location of the plurality of electrodes based on distortions in the electromagnetic field detected at a later second time; and
code for outputting a signal configured to cause simultaneous display of a first visualization of the reference location and a second visualization of the current location such that the current location is displayed relative to the reference location.
